(19) Europäisches Patentamt
European Patent Office
Office européen des brevets

(11) Veröffentlichungsnummer: **0 544 185 A1**

(12) **EUROPÄISCHE PATENTANMELDUNG**

(21) Anmeldenummer: **92119697.8**

(51) Int. Cl.5: **C07C 309/17**, B03D 1/00

(22) Anmeldetag: **19.11.92**

(30) Priorität: **27.11.91 DE 4138911**

(43) Veröffentlichungstag der Anmeldung: **02.06.93 Patentblatt 93/22**

(84) Benannte Vertragsstaaten: **PT**

(71) Anmelder: **Henkel Kommanditgesellschaft auf Aktien**
**Henkelstrasse 67**
**W-4000 Düsseldorf 13(DE)**

(72) Erfinder: **Köster, Rita**
**Taubenstrasse 7**
**W-4000 Düsseldorf 30(DE)**
Erfinder: **Schreck, Berthold, Dr.**
**Maurerstrasse 26**
**W-4000 Düsseldorf 30(DE)**
Erfinder: **Kluschanzoff, Harald, Dr.**
**Berta-von-Suttner-Strasse 41**
**W-4000 Düsseldorf-Hellerhof(DE)**

(54) **Verfahren zur Gewinnung von Mineralien aus nichtsulfidischen Erzen durch Flotation.**

(57) Mineralien lassen sich aus nichtsulfidischen Erzen durch Flotation anreichern, indem man als Sammler Sulfosuccinate der Formel **(Ia)** und/oder **(Ib)**,

$$R^1O-(CH_2CH_2O)_n-CO-CH_2CH(SO_3M)-COOM \quad (Ia)$$

$$R^2CO-O-(CH_2CH_2O)_m-CO-CH_2CH(SO_3M)-COOM \quad (Ib)$$

in der

| | |
|---|---|
| $R^1$ | für einen Hydroxyalkylrest mit 6 bis 22 Kohlenstoffatomen, |
| $R^2CO$ | für einen Acylrest mit 6 bis 22 Kohlenstoffatomen, |
| n | für 0 oder Zahlen von 1 bis 10, |
| m | für Zahlen von 1 bis 10 und |
| M | für Natrium oder Kalium steht, |

gegebenenfalls in Kombination mit weiteren anionischen und/oder nichtionischen Tensiden einsetzt.

EP 0 544 185 A1

## Gebiet der Erfindung

Die Erfindung betrifft neue Sulfosuccinate, ein Verfahren zu ihrer Herstellung sowie ein Verfahren zur Gewinnung von Mineralien aus nichtsulfidischen Erzen durch Flotation, bei dem man die neuen Sulfosuccinate, gegebenenfalls im Gemisch mit weiteren anionischen und/oder nichtionischen Tensiden als Sammler einsetzt.

## Stand der Technik

Zur Abtrennung von Wertmineralien von der Gangart ist die Flotation ein allgemein angewandtes Sortierverfahren, das der Aufbereitung von mineralischen Erzen dient. Üblicherweise wird hierbei das Erz zunächst zerkleinert und trocken, vorzugsweise aber naß vermahlen und in Wasser suspendiert. Im Anschluß wird ein Sammler, häufig in Verbindung mit weiteren Reagenzien, zu denen Schäumer, Regler, Drücker (Desaktivatoren) und/oder Beleber (Aktivatoren) gehören, zugegeben, der die Abtrennung der Wertminerale von den Gangartmineralien des Erzes bei der anschließenden Flotation unterstützt. Bevor in die Suspension Luft eingeblasen wird, um an ihrer Oberfläche Schaum zu erzeugen und die Flotation in Gang zu setzen, läßt man diese Reagenzien üblicherweise eine gewisse Zeit auf das feingemahlene Erz einwirken (Konditionieren). Der Sammler bewirkt eine Hydrophobierung der Oberfläche der Minerale, so daß ein Anhaften dieser Minerale an den während der Belüftung gebildeten Gasblasen stattfindet. Die Hydrophobierung der Mineralbestandteile erfolgt selektiv in der Weise, daß die unerwünschten Bestandteile des Erzes nicht an den Gasblasen anhaften und zurückbleiben, während der mineralhaltige Schaum abgestreift und weiterverarbeitet wird. Im umgekehrten Fall, bei der sogenannten indirekten Flotation, wird die Verunreinigung ausflotiert, während das Wertmineral zurückbleibt. Das Ziel der Flotation besteht darin, das Wertmineral der Erze in möglichst hoher Ausbeute zu gewinnen und dabei gleichzeitig eine möglichst gute Anreicherung des Wertminerals zu erhalten.

Bei der flotativen Aufbereitung nichtsulfidischer Erze werden als Sammler überwiegend anionische oder kationische Tenside eingesetzt. Diesen kommt die Aufgabe zu, an der Oberfläche der Wertmineralien oder der Verunreinigung möglichst selektiv zu adsorbieren, um eine hohe Anreicherung im Flotationskonzentrat sicherzustellen. Außerdem sollen die Sammler einen tragfähigen, aber nicht zu stabilen Flotationsschaum entwikkeln.

Die bei der Flotation nichtsulfidischer Erze häufig verwendeten Sammler, wie z. B. Fettsäuren, Alkylsulfosuccinate [**Aufbereitungstechnik, <u>26</u>, 632 (1985)**], Dicarbonsäureester [**EP 0 067 137 B1, DE 40 10 911, US 2,099,120**] oder Oleylsulfate [**DE-OS-1.029.761**], führen jedoch in vielen Fällen bei ökonomisch vertretbaren Sammlermengen nicht zu einem befriedigenden Flotationsergebnis.

Aufgabe der Erfindung war es daher, Sammlersysteme zu entwickeln, die frei von den geschilderten Nachteilen sind.

## Beschreibung der Erfindung

Gegenstand der Erfindung ist ein Verfahren zur Gewinnung von Mineralien aus nichtsulfidischen Erzen durch Flotation, bei dem man gemahlenes Erz mit Wasser zu einer Suspension mischt, in die Suspension in Gegenwart eines Reagenziensystems Luft einleitet und den entstandenen Schaum zusammen mit den darin enthaltenen flotierten Feststoffen abtrennt, und bei dem man als Sammler Sulfosuccinate der Formel **(Ia)** und/oder **(Ib)**,

$$R^1O-(CH_2CH_2O)_n-CO-CH_2\underset{\displaystyle SO_3M}{\underset{|}{CH}}-COOM \qquad \mathbf{(Ia)}$$

$$R^2CO-O-(CH_2CH_2O)_m-CO-CH_2\underset{\displaystyle SO_3M}{\underset{|}{CH}}-COOM \qquad \mathbf{(Ib)}$$

in der

| | |
|---|---|
| $R^1$ | für einen Hydroxyalkylrest mit 6 bis 22 Kohlenstoffatomen, |
| $R^2CO$ | für einen Acylrest mit 6 bis 22 Kohlenstoffatomen, |
| n | für 0 oder Zahlen von 1 bis 10, |
| m | für Zahlen von 1 bis 10 und |
| M | für Natrium oder Kalium steht, |

gegebenenfalls in Kombination mit weiteren anionischen und/oder nichtionischen Tensiden einsetzt.

Überraschenderweise wurde festgestellt, daß Sulfosuccinate der Formel **(Ia)** und/oder **(Ib)** in der Flotation von nichtsulfidischen Erzen eine äußerst geringe Schaumentwicklung zeigen, so daß ein übermäßiges Schäumen in den Flotationszellen vermieden werden kann. Die Erfindung schließt die Erkenntnis ein, daß die Sammler eine hohe Aktivität und Selektivität zeigen, die das praktisch quantitative Ausbringen der Mineralien mit gegenüber dem Stand der Technik vergleichsweise geringen Einsatzmengen möglich macht.

Sulfosuccinate der Formel **(Ia)**, in der n für 0 oder Zahlen von 1 bis 3 und/oder $R^1$ für einen Hydroxyalkylrest mit 12 bis 18 Kohlenstoffatomen steht sowie Sulfosuccinate der Formel **(Ib)**, in der $R^2CO$ für einen Acylrest mit 12 bis 18 Kohlenstoffatomen und 0 oder 1 Doppelbindung steht, zeichnen sich durch besonders vorteilhafte Sammlereigenschaften in der Flotation aus und sind daher bevorzugt.

Das erfindungsgemäße Verfahren erlaubt den Einsatz der Sulfosuccinate der Formel **(Ia)** und/oder **(Ib)** für die Gewinnung von Mineralien aus nichtsulfidischen Erzen durch Flotation allein oder in Gegenwart von weiteren anionischen und/oder nichtionischen Tensiden.

Unter **anionischen Tensiden** sind im Sinne der Erfindung Fettsäuren, Alkylsulfate, Alkylethersulfate, Alkylsulfosuccinate, Alkylsulfosuccinamate, Alkylbenzolsulfonate, Alkansulfonate, Petrolsulfonate, Acyllactylate, Sarcoside, Alkylphosphate, Alkyletherphosphate, Alkylasparaginsäuren und Ethercarbonsäuren zu verstehen. Bei allen diesen anionischen Tensiden handelt es sich um bekannte Verbindungen, deren Herstellung - sofern nicht anders angegeben - z.B. in **J.Falbe, U.Hasserodt (ed.), "Katalysatoren, Tenside und Mineralöladditive, Thieme Verlag, Stuttgart, 1978** oder **J.Falbe (ed.) "Surfactants in Consumer Products", Springer Verlag, Berlin, 1986** beschrieben ist.

Als Fettsäuren kommen hier vor allem die aus pflanzlichen oder tierischen Fetten und Ölen, beispielsweise durch Fettspaltung und gegebenenfalls Fraktionierung und/oder Trennung nach dem Umnetzverfahren, gewonnenen geradkettigen Fettsäuren der Formel **(II)** in Betracht,

$$R^3\text{-COOY} \qquad \text{(II)}$$

in der $R^3$ für einen aliphatischen Kohlenwasserstoffrest mit 12 bis 18 Kohlenstoffatomen und 0, 1, 2 oder 3 Doppelbindungen und Y für ein Alkali-, Erdalkalimetall oder einen Ammoniumrest steht. Eine besondere Bedeutung kommt hierbei den Natrium- und Kaliumsalzen der Öl- und der Tallölfettsäure zu.

Als Alkylsulfate eignen sich die wasserlöslichen Salze von Schwefelsäurehalbestern von Fettalkoholen der Formel **(III)**,

$$R^4\text{-O-SO}_3Z \qquad \text{(III)}$$

in der $R^4$ für einen linearen oder verzweigten Alkylrest mit 8 bis 22, vorzugsweise 12 bis 18 Kohlenstoffatomen und Z für ein Alkalimetall oder einen Ammoniumrest steht.

Als Alkylethersulfate eignen sich die wasserlöslichen Salze von Schwefelsäurehalbestern von Fettalkoholpolyglycolethern der Formel **(IV)**,

$$R^5\text{-}(\text{OCH}_2\underset{\substack{|\\R^6}}{\text{CH}})_n\text{OSO}_3Z \qquad \text{(IV)}$$

in der $R^5$ für einen linearen oder verzweigten Alkylrest mit 8 bis 22, vorzugsweise 12 bis 18 Kohlenstoffatomen, $R^6$ für Wasserstoff oder eine Methylgruppe und n für 1 bis 30, vorzugsweise 2 bis 15 steht und Z die oben angegebene Bedeutung besitzt.

Als Alkylsulfosuccinate eignen sich Sulfobernsteinsäuremonoester von Fettalkoholen der Formel **(V)**,

$$R^7\text{-OOC-CH}_2\text{-CH(SO}_3\text{Z)-COOZ} \quad (V)$$

in der $R^7$ für einen linearen oder verzweigten Alkylrest mit 8 bis 22, vorzugsweise 12 bis 18 Kohlenstoffatomen steht und Z die oben angegebene Bedeutung besitzt.

Als Alkylsulfosuccinamate eignen sich Sulfobernsteinsäuremonoamide von Fettaminen der Formel **(VI)**,

$$R^8\text{-NH-OC-CH}_2\text{-CH(SO}_3\text{Z)-COOZ} \quad (VI)$$

in der $R^8$ für einen linearen oder verzweigten Alkylrest mit 8 bis 22, vorzugsweise 12 bis 18 Kohlenstoffatomen steht und Z die oben angegebene Bedeutung besitzt.

Als Alkylbenzolsulfonate eignen sich Substanzen der Formel **(VII)**,

**$R^9\text{-}C_6H_4\text{-}SO_3Z$ (VII)**

in der $R^9$ für einen geradkettigen oder verzweigten Alkylrest mit 4 bis 16, vorzugsweise 8 bis 12 Kohlenstoffatomen steht und Z die oben angegebene Bedeutung besitzt.

Als Alkansulfonate eignen sich Substanzen der Formel **(VIII)**,

**$R^{10}\text{-}SO_3Z$ (VIII)**

in der $R^{10}$ für einen geradkettigen oder verzweigten Alkylrest mit 12 bis 18 Kohlenstoffatomen steht und Z die oben angegebene Bedeutung besitzt.

Als Petrolsulfonate eignen sich Substanzen, die man durch Umsetzung von Schmierölfraktionen mit Schwefeltrioxid oder Oleum und anschließende Neutralisation mit Natronlauge erhält. Hier kommen insbesondere solche Produkte in Betracht, in denen die Kohlenwasserstoffreste überwiegend Kettenlängen von 8 bis 22 Kohlenstoffatomen aufweisen.

Als Acyllactylate eignen sich Substanzen der Formel **(IX)**,

$$R^{11}\text{-CO-O-CH(CH}_3\text{)-COOZ} \quad (IX)$$

in der $R^{11}$ für einen aliphatischen, cycloaliphatischen oder alicyclischen, gegebenenfalls mit Hydroxylgruppen substituierten Kohlenwasserstoffrest mit 7 bis 23 Kohlenstoffatomen und 0, 1, 2 oder 3 Doppelbindungen steht und Z die oben angegebene Bedeutung besitzt. Die Herstellung und Verwendung der Acyllactylate in der Flotation ist in der Deutschen Patentanmeldung **DE 32 38 060 A1** beschrieben.

Als Sarcoside eignen sich Substanzen der Formel **(X)**,

$$R^{12}\text{-CO-N(CH}_3\text{)-CH}_2\text{-COOH} \quad (X)$$

in der $R^{12}$ für einen aliphatischen Kohlenwasserstoffrest mit 12 bis 22 Kohlenstoffatomen und 0, 1, 2 oder 3 Doppelbindungen steht.

Als Alkylphosphate und Alkyletherphosphate eignen sich Substanzen der Formeln **(XI)** und **(XII)**,

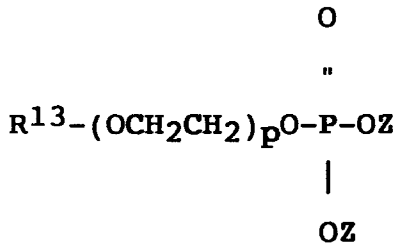

**(XI)**

und

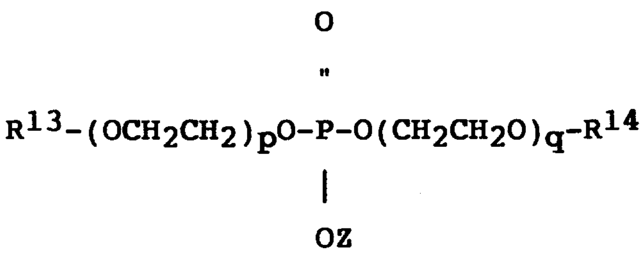

**(XII)**

in der $R^{13}$ und $R^{14}$ unabhängig voneinander für einen Alkyl- oder Alkenylrest mit 8 bis 22 Kohlenstoffatomen und p und q im Falle der Alkylphosphate für 0, im Falle der Alkyletherphosphate für Zahlen von 1 bis 15 stehen und Z die oben angegebene Bedeutung besitzt.

Werden die Sulfosuccinate im Sinne der Erfindung im Gemisch mit Alkylphosphaten oder Alkyletherphosphaten eingesetzt, können die Phosphate als Mono- oder Diphosphate vorliegen. Vorzugsweise werden in diesem Fall Gemische aus Mono- und Dialkylphosphaten eingesetzt, wie sie bei der technischen Herstellung solcher Verbindungen anfallen.

Unter Alkylasparaginsäuren sind Verbindungen der Formel **(XIII)** zu verstehen,

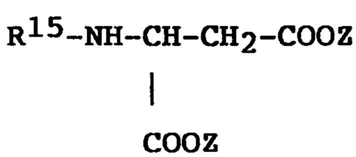

**(XIII)**

in der $R^{15}$ für einen Alkyl- oder Alkenylrest mit 8 bis 22 Kohlenstoffatomen steht und Z die oben angegebene Bedeutung besitzt.

Als Ethercarbonsäuren kommen schließlich Verbindungen der Formel **(XIV)** in Betracht,

$R^{16}O\text{-}(CH_2CH_2O)_n\text{-}CH_2\text{-}COOZ$ **(XIV)**

in der $R^{16}$ für einen Alkyl- oder Alkenylrest mit 8 bis 22 Kohlenstoffatomen und n für 0 oder Zahlen von 1 bis 10 steht und Z die oben angegebene Bedeutung besitzt.

Unter **nichtionischen Tensiden** sind im Sinne der Erfindung Fettalkoholpolyglycolether, Alkylphenolpolyglycolether, Fettsäurepolyglycolester, Fettsäureamidpolyglycolether, Fettaminpolyglycolether, Mischether, Hydroxymischether und Alkylglykoside zu verstehen. Bei allen diesen nichtionischen Tensiden handelt es sich um bekannte Verbindungen, deren Herstellung - sofern nicht anders angegeben - z.B. in **J.Falbe, U.Hasserodt (ed.), "Katalysatoren, Tenside und Mineralöladditive, Thieme Verlag, Stuttgart, 1978** oder **J.Falbe (ed.) "Surfactants in Consumer Products", Springer Verlag, Berlin, 1986** beschrieben ist.

Als Fettalkoholpolyglycolether eignen sich Anlagerungsprodukte von durchschnittlich n Mol Ethylen- und/oder Propylenoxid an Fettalkohole, die der Formel **(XV)** folgen,

$$R^{17}(OCH_2\underset{\underset{R^6}{|}}{C}H)_nOH \qquad (XV)$$

in der $R^{17}$ für einen linearen oder verzweigten Alkylrest mit 8 bis 22, vorzugsweise 12 bis 18 Kohlenstoffatomen, $R^6$ für Wasserstoff oder eine Methylgruppe und n für Zahlen von 1 bis 30, vorzugsweise 2 bis 15 steht.

Als Alkylphenolpolyalycolether eignen sich Anlagerungsprodukte von durchschnittlich n Mol Ethylen- und/oder Propylenglycol an Alkylphenole, die der Formel **(XVI)** folgen,

$$R^{18}\text{-}C_6H_4\text{-}(OCH_2\underset{\underset{R^6}{|}}{C}H)_nOH \qquad (XVI)$$

in der $R^{18}$ für einen Alkylrest mit 4 bis 15, vorzugsweise 8 bis 10 Kohlenstoffatomen steht und $R^6$ und n die oben angegebenen Bedeutungen besitzen.

Als Fettsäurepolyglycolester eignen sich Anlagerungsprodukte von durchschnittlich n Mol Ethylen- und/oder Propylenoxid an Fettsäuren, die der Formel **(XVII)** folgen,

$$R^{19}\text{-}CO(OCH_2\underset{\underset{R^6}{|}}{C}H)_nOH \qquad (XVII)$$

in der $R^{19}$ für einen aliphatischen Kohlenstoffrest mit 5 bis 21 Kohlenstoffatomen und 0, 1, 2 oder 3 Doppelbindungn steht und $R^6$ und n die oben angegebenen Bedeutungen besitzen.

Als Fettsäureamidpolvglycolether eignen sich Anlagerungsprodukte von durchschnittlich n Mol Ethylen- und/oder Propylenoxid an Fettsäureamide, die der Formel **(XVIII)** folgen,

$$R^{20}\text{-}CO\text{-}NH(OCH_2\underset{\underset{R^6}{|}}{C}H)_nOH \qquad (XVIII)$$

in der $R^{20}$ für einen aliphatischen Kohlenwasserstoffrest mit 5 bis 21 Kohlenstoffatomen und 0, 1, 2 oder 3 Doppelbindungen steht und $R^6$ und n die oben angegebenen Bedeutungen besitzen.

Als Fettaminpolyglycolether eignen sich Anlagerungsprodukte von durchschnittlich n Mol Ethylen- und/oder Propylenoxid an Fett-amine, die der Formel **(XIX)** folgen,

$$R^{21}-NH(OCH_2\underset{\displaystyle R^6}{\underset{|}{C}}H)_nOH \qquad (XIX)$$

in der $R^{21}$ für einen Alkylrest mit 6 bis 22 Kohlenstoffatomen steht und $R^6$ und n die oben angegebenen Bedeutungen besitzen.

Als Mischether eignen sich Umsetzungsprodukte von Fettalkoholpolyglycolethern mit Alkylchloriden der Formel **(XX)**,

$$R^{22}-(OCH_2\underset{\displaystyle R^6}{\underset{|}{C}}H)_n-O-R^{23} \qquad (XX)$$

in der $R^{22}$ für einen aliphatischen Kohlenstoffrest mit 6 bis 22 Kohlenstoffatomen und 0, 1, 2 oder 3 Doppelbindungen, $R^{23}$ für einen Alkylrest mit 1 bis 4 Kohlenstoffatomen oder einen Benzylrest steht und $R^6$ und n die oben angegebenen Bedeutungen besitzen.

Als Hydroxymischether eignen sich Substanzen der Formel **(XXI)**,

$$R^{24}-\underset{\displaystyle OH}{\underset{|}{C}}H-CH_2-(OCH_2\underset{\displaystyle R^6}{\underset{|}{C}}H)_n-O-R^{25} \qquad (XXI)$$

in der $R^{24}$ für einen Alkylrest mit 6 bis 16 Kohlenstoffatomen, $R^{25}$ für einen Alkylrest mit 1 bis 4 Kohlenstoffatomen oder einen Benzylrest steht und $R^6$ und n die oben angegebenen Bedeutungen besitzen. Die Herstellung der Hydroxymischether ist in der Deutschen Patentanmeldung **DE 37 23 323 A1** beschrieben.

Als Alkylglykoside eignen sich Substanzen der Formel **(XXII)**,

$$R^{26}-O-(G)_x \qquad (XXII)$$

in der G ein Symbol für eine Glykose-Einheit darstellt, die sich von einem Zucker mit 5 oder 6 Kohlenstoffatomen ableitet, x für eine Zahl zwischen 1 und 10 und $R^{26}$ für einen aliphatischen Kohlenwasserstoffrest mit 6 bis 22 Kohlenstoffatomen und 0, 1, 2 oder 3 Doppelbindungen steht. Vorzugsweise steht G für eine Glucoseeinheit und x für Zahlen von 1,1 bis 1,6. Die Herstellung der Alkylglykoside ist beispielsweise in der Deutschen Patentanmeldung **DE 37 23 826 A1** beschrieben.

Die Gemische der erfindungsgemäßen Sulfosuccinate mit den anionischen und/oder nichtionischen Tensiden können einen Gehalt von 5 bis 95 Gew.-%, vorzugsweise 10 bis 60 Gew.-% der Verbindungen der Formel **(Ia)** und/oder **(Ib)** aufweisen. Besonders vorteilhafte Ergebnisse werden mit Gemischen erzielt, die neben den erfindungsgemäßen Sulfosuccinaten Fettsäuren der Formel **(II)** und/oder Alkylsulfosuccinate der Formel **(V)** enthalten.

Zur Erzielung wirtschaftlich brauchbarer Ergebnisse bei der Flotation nichtsulfidischer Erze müssen die erfindungsgemäßen Sulfosuccinate in gewissen Mindestmengen eingesetzt werden. Es darf aber auch eine Höchstmenge nicht überschritten werden, da sonst die Selektivität gegenüber den Wertmineralien abnimmt. Die Mengen, in denen die erfindungsgemäßen Sulfosuccinate bzw. deren Gemische mit weiteren anionischen und/oder nichtionischen Tensiden eingesetzt werden, hängen von der Art der zu flotierenden Erze und von deren Gehalt an Wertmineralien ab. Demzufolge können die jeweils notwendigen Einsatzmengen in weiten Grenzen schwanken. Im allgemeinen werden die Sulfosuccinate bzw. deren Gemische mit anionischen und/oder nichtionischen Tensiden in Mengen von 20 bis 2000, vorzugsweise 50 bis 1000 g pro Tonne Roherz eingesetzt.

Das erfindungsgemäße Verfahren schließt die Mitverwendung von für die Flotation üblichen Reagenzien wie beispielsweise Schäumern, Reglern, Aktivatoren, Desaktivatoren usw. ein. Die Durchführung der Flotation erfolgt unter den Bedingungen der Verfahren des Standes der Technik. In diesem Zusammenhang sei auf die folgenden Literaturstellen zum technologischen Hintergrund der Erzaufbereitung verwiesen : **H.Schubert, "Aufbereitung fester mineralischer Stoffe", Leipzig, 1967; D.B. Puchas (Ed.), "Solid/liquid separation equipment scale-up", Croydon, 1977; E.S.Perry, C.J.VanOss, E.Grushka (Ed.), "Separation and Purification Methods", New York, 1973 - 1978.**

Bevorzugt werden in der Flotation nichtsulfidische Erze von Salzmineraltyp, insbesondere Apatit, eingesetzt.

Ein weiterer Gegenstand der Erfindung betrifft Sulfosuccinate der Formel **(Ia)** und/oder **(Ib)**

$$R^1O-(CH_2CH_2O)_n-CO-CH_2\underset{\displaystyle SO_3M}{\underset{|}{CH}}-COOM \qquad (Ia)$$

$$R^2CO-O-(CH_2CH_2O)_m-CO-CH_2\underset{\displaystyle SO_3M}{\underset{|}{CH}}-COOM \qquad (Ib)$$

in der

| | |
|---|---|
| $R^1$ | für einen Hydroxyalkylrest mit 6 bis 22 Kohlenstoffatomen, |
| $R^2CO$ | für einen Acylrest mit 6 bis 22 Kohlenstoffatomen, |
| n | für 0 oder Zahlen von 1 bis 10, |
| m | für Zahlen von 1 bis 10 und |
| M | für Natrium oder Kalium steht, |

Ein weiterer Gegenstand der Erfindung betrifft ein Verfahren zur Herstellung von Sulfosuccinaten der Formel **(Ia)** und/oder **(Ib)**,

$$R^1O-(CH_2CH_2O)_n-CO-CH_2\underset{\displaystyle SO_3M}{\underset{|}{CH}}-COOM \qquad (Ia)$$

$$R^2CO-O-(CH_2CH_2O)_m-CO-CH_2\underset{\displaystyle SO_3M}{\underset{|}{CH}}-COOM \qquad (Ib)$$

in der

| | |
|---|---|
| $R^1$ | für einen Hydroxyalkylrest mit 6 bis 22 Kohlenstoffatomen, |
| $R^2CO$ | für einen Acylrest mit 6 bis 22 Kohlenstoffatomen, |
| n | für 0 oder Zahlen von 1 bis 10, |
| m | für Zahlen von 1 bis 10 und |
| M | für Natrium oder Kalium steht, |

das sich dadurch auszeichnet, daß man Maleinsäureanhydrid mit Hydroxyalkoholen bzw. deren (Poly-)ethylenglycolethern der Formel **(Ic)** und/oder Fettsäure(poly)ethylenglycolestern der Formel **(Id)**,

**$R^1O-(CH_2CH_2O)_nH$ (Ic)**

**$R^2CO-O-(CH_2CH_2O)_mH$ (Id)**

in der $R^1$, $R^2CO$, n und m die oben angegebene Bedeutung besitzen, zu den entsprechenden Maleinsäurehalbestern umsetzt und an diese in an sich bekannter Weise Natrium- oder Kaliumbisulfit anlagert.

Zur selektiven Herstellung von Maleinsäurehalbestern empfiehlt es sich bekanntlich, die Reaktion in Gegenwart eines sauren Katalysators mit äquimolaren Mengen der Einsatzstoffe bei einer Temperatur von 70 bis 100 °C durchzuführen. Die Addition von Bisulfit an die Doppelbindung des Maleinsäurehalbesters kann in wäßriger und/oder methanolischer Lösung bei Temperaturen von ebenfalls 70 bis 100 °C, gegenenfalls unter Druck durchgeführt werden. In diesem Zusammenhang sei auf die einschlägige Literatur verwiesen, in der Methoden zur allgemeinen Herstellung von Sulfosuccinaten umfassend referiert werden [**Fette, Seifen, Anstrichmitt., 65, 748 (1963), Tens. Surf.Det., 11, 202 (1974), HAPPI 18, No.3, 68 (1981)**].

Als Alkoholkomponenten zur Herstellung der Sulfosuccinate kommen Hydroxyalkohole bzw. deren (Poly-)ethylenglycolether der Formel **(Ic)** und/oder Fettsäure(poly)ethylenglycolester der Formel **(Id)** in Betracht.

Typische Beispiele für Alkoholkomponenten der Formel **(Ic)** sind 2-Hydroxycapronalkohol, 2-Hydroxycaprylalkohol, 2-Hydroxycaprinalkohol, 2-Hydroxylaurylalkohol, 2-Hydroxymyristylalkohol, 2-Hydroxycetylalkohol, 2-Hydroxystearylalkohol, 12-Hydroxystearylalkohol, 2-Hydroxyarachylalkohol und 2-Hydroxybehenylalkohol sowie deren Ethylenoxidanlagerungsprodukte. Die 2-Hydroxyalkohole können beispielsweise durch Ringöffnung von alpha-Olefinepoxiden mit Wasser erhalten werden; 12-Hydroxystearylalkohol stellt das Produkt der Hydrierung von Ricinolylalkohol dar. Die Anlagerung von Ethylenoxid an die Hydroxyalkohole unter Bildung der entsprechenden (Poly-)ethylenglycolether kann in an sich bekannter Weise durchgeführt werden. Da primäre Hydroxyfunktionen in der Ethoxylierung weitaus rascher abreagieren als sekundäre, stellen die sich von den Hydroxyalkoholen ableitenden (Poly-)glycolether überwiegend Addukte des Ethylenoxids an die primäre OH-Funktion dar. Aus anwendungstechnischen Gründen bevorzugt sind Hydroxyalkohole bzw. deren Addukte mit 1 bis 3 Ethylenoxideinheiten, die 12 bis 18 Kohlenstoffatome in der Fettkette aufweisen.

Typische Beispiele für Alkoholkomponenten der Formel **(Id)** sind Monoester von gesättigten oder ungesättigten Fettsäuren mit 6 bis 22 Kohlenstoffatomen und 0, 1, 2 oder 3 Doppelbindungen mit Ethylenglycol oder höheren Kondensationsprodukten des Ethylenoxids, die noch über eine freie Hydroxylgruppe verfügen. Typische Beispiele sind die Monester der Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, Arachinsäure, Gadoleinsäure, Behensäure und Erucasäure mit Ethylen-, Diethylen- oder Triethylenglycol. Polyglycolester der Formel **(Id)** sind dabei nicht nur durch direkte Umsetzung der Fettsäuren mit den Glycolen erhältlich, sondern können auch durch Ethoxylierung der Fettsäuren hergestellt werden. Bevorzugt sind Monoester von Fettsäuren mit 12 bis 18 Kohlenstoffatomen und 0 oder 1 Doppelbindung mit Mono-, Di- oder Triethylenglycol.

Die folgenden Beispiele sollen den Gegenstand der Erfindung näher erläutern, ohne ihn darauf einzuschränken.

## Beispiele

### *I. Eingesetzte Sammler*

a) Erfindungsgemäße Sammler

A1) Sulfobernsteinsäure-(2-hydroxyoctadecyl)-monoester-di-Na-Salz;
Erhältlich durch Anlagerung von Natriumbisulfit an ein Umsetzungsprodukt von Maleinsäureanhydrid (MSA) mit 2-Hydroxy-stearylalkohol.

A2) Sulfobernsteinsäure-(2-hydroxytridecyl)-triethylenglycolmonoester-di-Na-Salz;
Erhältlich durch Anlagerung von Natriumbisulfit an ein Umsetzungsprodukt von MSA mit 2-Hydroxytridecanol-3 EO-Addukt.

A3) Sulfobernsteinsäure-(octadec-9-enoyl)-monoethylenglycolmonoester-di-Na-Salz;
Erhältlich durch Anlagerung von Natriumbisulfit an ein Umsetzungsprodukt von MSA mit Ölsäuremonoethylenglycolmonoester.

A4) Sulfobernsteinsäure-(octadecanoyl)-monoethylenglycolmonoester-di-Na-Salz;
Erhältlich durch Anlagerung von Natriumbisulfit an ein Kondensationsprodukt von MSA mit Stearinsäure-monoethylenglycolmonoester.

b) Vergleichssammler

B1) Ölsäure-Natriumsalz
B2) Maleinsäure-(octadecanoyl)-monoethylenglycol-monoesterNa-Salz;
Erhältlich durch Umsetzung von MSA mit Stearinsäuremonoethylenglycol-monoester **[DE 40 10 911 A1]**.
B3) Talgalkylsulfosuccinat-di-Natriumsalz

***II. Flotationsversuche in der Denver–Zelle***

Als Flotationsaufgabe wurde ein geringhaltiges Apatiterz mit einem hohen Anteil silicatischer Gangart verwendet, das folgende Zusammensetzung besaß (Durchschnittsanalyse):

| | |
|---|---|
| $P_2O_5$ | 4 Gew.-% |
| $SiO_2$ | 38 Gew.-% |
| CaO | 10 Gew.-% |
| MgO | 16 Gew.-% |

Die Flotationsaufgabe wies folgende Korngrößenverteilung auf:

| | |
|---|---|
| - 40 µm | 20,8 Gew.-% |
| 40 - 100 µm | 19,0 Gew.-% |
| 100 - 200 µm | 29,1 Gew.-% |
| 200 - 500 µm | 26,5 Gew.-% |
| > 500 µm | 4,6 Gew.-% |

Flotiert wurde in einer Denver-Laborflotationsmaschine Typ D1. Die Vorflotation wurde in einer 2-l-Zelle, die Reinigungflotation (1 bis 3 Stufen) in einer 1-l-Zelle durchgeführt. Die Trübedichte bei der Vorflotation betrug 500 g/l. Die Flotation erfolgte in weichem Wasser mit einem Gehalt an Calciumionen von 6 mg/l.

Als Drücker wurde ein Phenol-Formaldehyd-Kondensat (Suspendol(R) PPK, Fa Henkel KGaA, Düsseldorf, FRG) in einer Dosierung von 200 g/t eingesetzt. Die Konditionierungszeit betrug für Drücker und Sammler jeweils 5 min.

Die Konditionierung wurde bei einer Geschwindigkeit von 1100 UpM, die Flotation bei 1100 UpM (2-l-Zelle) bzw. 1000 UpM (1-l-Zelle) über jeweils 4 min durchgeführt. Die Sammler A1 bis A4 wurden alleine oder in Abmischung mit B1 im Gewichtsverhältnis 1 : 1 eingesetzt.

Die Ergebnisse der Flotation sind in Tab.1 und 2 zusammengefaßt.

Tab.1a: Flotation in der Denverzelle (I)

| Bsp. | EM g/t | Sammler | | FS | Gehalt (Gew.-% | | | |
|---|---|---|---|---|---|---|---|---|
| | | A | B | | $P_2O_5$ | $SiO_2$ | MgO | CaO |
| 1 | 80 | A1 | | rt<br>ct<br>conc | 0,91<br>6,29<br>28,67 | 42,5<br>23,6<br>2,8 | 18,0<br>12,0<br>3,6 | 4,9<br>26,5<br>51,5 |
| 2 | 60 | A2 | | rt<br>ct<br>conc | 0,92<br>2,95<br>26,55 | 42,6<br>32,2<br>5,2 | 19,5<br>15,6<br>5,0 | 5,4<br>16,8<br>47,4 |
| 3 | 60 | A2 | B1 | rt<br>ct<br>conc | 0,47<br>1,76<br>27,39 | 43,8<br>31,2<br>2,4 | 20,2<br>16,6<br>3,2 | 3,6<br>17,4<br>50,8 |
| 4 | 40 | A3 | | rt<br>ct<br>conc | 0,97<br>3,40<br>31,04 | 42,3<br>27,2<br>2,8 | 18,7<br>14,4<br>3,1 | 5,9<br>23,0<br>50,9 |
| 5 | 200 | A4 | | rt<br>ct<br>conc | 0,76<br>9,20<br>32,73 | 41,3<br>20,8<br>3,2 | 19,4<br>12,2<br>2,6 | 6,0<br>29,3<br>50,7 |

Forts.Tab.1a: Flotation in der Denverzelle (I)

| Bsp. | EM g/t | Sammler | | FS | Gehalt (Gew.-% | | | |
|---|---|---|---|---|---|---|---|---|
| | | A | B | | $P_2O_5$ | $SiO_2$ | MgO | CaO |
| 6 | 140 | A4 | B1 | rt | 0,15 | 44,5 | 20,6 | 2,8 |
| | | | | ct | 1,63 | 25,6 | 14,6 | 20,6 |
| | | | | conc | 29,35 | 1,3 | 2,6 | 51,3 |
| V1 | 142 | | B1 | rt | 0,37 | 44,2 | 18,0 | 3,0 |
| | | | | ct | 1,31 | 35,1 | 16,7 | 11,6 |
| | | | | conc | 24,21 | 3,9 | 4,3 | 48,3 |
| V2 | 300 | | B2 | rt | 3,14 | 38,3 | 16,8 | 9,9 |
| | | | | ct | 6,79 | 33,1 | 18,2 | 15,4 |
| | | | | conc | 7,92 | 36,9 | 19,2 | 15,8 |

Tab.2a

| Flotation in der Denverzelle (II) | | | | |
|---|---|---|---|---|
| Bsp | FS | Ausbringen (Gew.-%) | | RS |
| | | Masse | $P_2O_5$ | |
| 1 | rt<br>ct<br>conc | 84,9<br>6,3<br>8,9 | 20,7<br>10,7<br>68,6 | 2 |
| 2 | rt<br>ct<br>conc | 82,7<br>8,5<br>8,8 | 22,7<br>7,5<br>69,8 | 4 |
| 3 | rt<br>ct<br>conc | 79,4<br>9,9<br>10,7 | 10,8<br>5,0<br>84,2 | 4 |
| 4 | rt<br>ct<br>conc | 87,2<br>4,3<br>8,5 | 23,3<br>4,1<br>72,6 | 2 |
| 5 | rt<br>ct<br>conc | 88,6<br>4,5<br>6,9 | 20,2<br>12,3<br>67,5 | 1 |
| 6 | rt<br>ct<br>conc | 77,0<br>12,4<br>10,6 | 3,4<br>5,9<br>90,7 | 3 |

Tab.6b

| Hämatit, Probe II: Konzentrationen auf Mühlenaufgabe bezogen Prozentangaben als Gew.-% | | | | | | |
|---|---|---|---|---|---|---|
| Bsp. | GK min | Eisenkonzentrat | | | | Ausbringen |
| | | Menge % | Fe % | $SiO_2$ % | P % | Fe % |
| 12 | 0 | 32,8 | 69,8 | 3,1 | 0,012 | 57,4 |
| 13 | 0 | 31,8 | 68,8 | 2,7 | 0,013 | 56,1 |
| 14 | 0 | 33,4 | 68,5 | 2,3 | 0,012 | 60,2 |
| 15 | 0 | 33,5 | 68,4 | 2,4 | 0,012 | 60,1 |
| 16 | 0 | 31,7 | 67,7 | 3,2 | 0,013 | 56,5 |
| 17 | 0 | 31,5 | 68,2 | 3,1 | 0,011 | 55,2 |
| 18 | 0 | 30,9 | 68,1 | 3,4 | 0,010 | 55,1 |
| 19 | 0 | 31,0 | 67,5 | 3,5 | 0,010 | 55,3 |
| 20 | 0 | 31,9 | 68,2 | 3,5 | 0,014 | 57,3 |
| V2 | 0 | 32,4 | 70,2 | 2,5 | 0,021 | 57,5 |

### *III. Flotation in der modifizierten Hallimondröhre*

Für die Mikroflotation wurde schwedisches Apatiterz verwendet, das die folgende Zusammensetzung aufwies (Durchschnittsanalyse):

| | |
|---|---|
| $P_2O_5$ | 20,1 Gew.-% |
| $Fe_2O_3$ | 6,3 Gew.-% |
| $SiO_2$ | 32,2 Gew.-% |
| CaO | 34,2 Gew.-% |
| $Al_2O_3$ | 5,1 Gew.-% |

Das Erz lag bereits fein gemahlen in der Trübe vor. Es wurde naß gesiebt und eine Korngrößenfraktion von 50 bis 100 µm als Aufgabe für die Flotation ausgewählt.

Als Flotationszelle wurde eine modifizierte Hallimondröhre mit einem Volumen von 165 ml und einer Rührgeschwindigkeit von 400 UpM verwendet. Konditioniert und flotiert wurden jeweils 2 g Erz (Trockengehalt) bei pH 10 (mit Natriumhydroxidösung eingestellt) in hartem Wasser (20 °d, nur Calciumionen). Die Konditionierzeiten betrugen für das Härtewasser 5 min und für den Sammler 10 min. Die Flotation wurde über einen Zeitaum von 2 min durchgeführt.

Die Sammler A2 und A3 wurden entweder alleine oder in Abmischung mit den Co-Sammlern B1, B2 oder B3 im Gewichtsverhältnis 1 : 1 eingesetzt. Die Ergebnisse sind in Tab.3 zusammengefaßt.

Tab.3: Flotation in der modifizierten Hallimond-Röhre

Prozentangaben als Gew.-%

| Bsp | ES g/t | Sammler | | Ausbringen (%) | |
|---|---|---|---|---|---|
| | | S1 | S2 | Masse | $P_2O_5$ |
| 7 | 100 | A2 | | 18 | 38 |
| 8 | 100 | A3 | | 18 | 37 |
| 9 | 200 | A3 | | 35 | 67 |
| 10 | 100 | A2 | B3 | 18 | 39 |
| 11 | 100 | A3 | B3 | 18 | 37 |
| V3 | 200 | | B1 | 16 | 32 |
| V4 | 200 | | B2 | 3 | 0 |
| V5 | 200 | | B3 | 15 | 50 |

Forts.Tab.3: Flotation in der modifizierten Hallimond-Röhre

Prozentangaben als Gew.-%

| Bsp. | ES g/t | Sammler | | Konzentratgehalt (%) | | |
|---|---|---|---|---|---|---|
| | | A | B | $P_2O_5$ | $Fe_2O_3$ | $SiO_2$ |
| 7 | 100 | A2 | | 41 | 1,3 | 2,8 |
| 8 | 100 | A3 | | 41 | 1,2 | 2,4 |
| 9 | 200 | A3 | | 38 | 1,6 | 3,2 |
| 10 | 100 | A2 | B3 | 42 | 1,0 | 1,7 |
| 11 | 100 | A3 | B3 | 40 | 1,3 | 2,2 |
| V3 | 200 | | B1 | 39 | 1,7 | 4,1 |
| V4 | 200 | | B2 | - | - | - *) |
| V5 | 200 | | B3 | 38 | 1,7 | 3,9 |

*) Zu geringer Austrag, keine Angabe möglich.

## Patentansprüche

**1.** Verfahren zur Gewinnung von Mineralien aus nichtsulfidischen Erzen durch Flotation, bei dem man gemahlenes Erz mit Wasser zu einer Suspension mischt, in die Suspension in Gegenwart eines Reagenziensystems Luft einleitet und den entstandenen Schaum zusammen mit den darin enthaltenen flotierten Feststoffen abtrennt, **dadurch gekennzeichnet,** daß man als Sammler Sulfosuccinate der Formel **(Ia)** und/oder **(Ib)**,

$$R^1O-(CH_2CH_2O)_n-CO-CH_2\underset{\displaystyle SO_3M}{\underset{|}{CH}}-COOM \qquad (Ia)$$

$$R^2CO-O-(CH_2CH_2O)_m-CO-CH_2\underset{\displaystyle SO_3M}{\underset{|}{CH}}-COOM \qquad (Ib)$$

in der

| | |
|---|---|
| $R^1$ | für einen Hydroxyalkylrest mit 6 bis 22 Kohlenstoffatomen, |
| $R^2CO$ | für einen Acylrest mit 6 bis 22 Kohlenstoffatomen, |
| n | für 0 oder Zahlen von 1 bis 10, |
| m | für Zahlen von 1 bis 10 und |
| M | für Natrium oder Kalium steht, |

gegebenenfalls in Kombination mit weiteren anionischen und/oder nichtionischen Tensiden einsetzt.

**2.** Verfahren nach Anspruch 1, **dadurch gekennzeichnet,** daß man Sulfosuccinate der Formel **(Ia)** einsetzt, in der n für 0 steht.

**3.** Verfahren nach Anspruch 1, **dadurch gekennzeichnet,** daß man Sulfosuccinate der Formel **(Ia)** einsetzt, in der n für Zahlen von 1 bis 3 steht.

**4.** Verfahren nach mindestens einem der Ansprüche 1 bis 3, **dadurch gekennzeichnet,** daß man Sulfosuccinate der Formel **(Ia)** einsetzt, in der $R^1$ für einen Hydroxyalkylrest mit 12 bis 18 Kohlenstoffatomen steht.

**5.** Verfahren nach mindestens einem der Ansprüche 1 bis 3, **dadurch gekennzeichnet,** daß man Sulfosuccinate der Formel **(Ib)** einsetzt, in der $R^2CO$ für einen Acylrest mit 12 bis 18 Kohlenstoffatomen und 0 oder 1 Doppelbindung steht.

**6.** Verfahren nach mindestens einem der Ansprüche 1 bis 5, **dadurch gekennzeichnet,** daß man Sulfosuccinate der Formel **(Ia)** und/oder **(Ib)** in Abmischung mit anionischen Tensiden einsetzt, die ausgewählt sind aus der Gruppe, die von Fettsäuren, Alkylsulfaten, Alkylethersulfaten, Alkylsulfosuccinaten, Alkylsulfosuccinamaten, Alkylbenzolsulfonaten, Alkansulfonaten, Petrolsulfonaten, Acyllactylaten, Sarcosiden, Alkylphosphate, Alkyletherphosphaten, Alkylasparaginsäuren und Ethercarbonsäuren gebildet wird.

**7.** Verfahren nach mindestens einem der Ansprüche 1 bis 5, **dadurch gekennzeichnet,** daß man Sulfosuccinate der Formel **(Ia)** und/oder **(Ib)** in Abmischung mit nichtionischen Tensiden einsetzt, die ausgewählt sind aus der Gruppe, die von Fettalkoholpolyglycolethern, Alkylphenolpolyglycolethern, Fettsäurepolyglycolestern, Fettsäureamidopolyglycolethern, Fettaminpolyglycolethern, Mischethern, Hydroxymischethern und Alkylglycosiden gebildet wird.

**8.** Verfahren nach mindestens einem der Ansprüche 1 bis 7, **dadurch gekennzeichnet,** daß man Gemische einsetzt, die einen Anteil an Sulfosuccinaten der Formel **(Ia)** und/oder **(Ib)** von 5 bis 95 Gew.-% - bezogen auf die Mischung - aufweisen.

**9.** Verfahren nach mindestens einem der Ansprüche 1 bis 8, **dadurch gekennzeichnet,** daß man die Sulfosuccinate der Formel **(Ia)** und/oder **(Ib)** alleine oder in Abmischung mit anionischen und/oder nichtionischen Tensiden in Mengen von 20 bis 2000 g/t Roherz einsetzt.

**10.** Verfahren nach mindestens einem der Ansprüche 1 bis 9, **dadurch gekennzeichnet,** daß man die Flotation mit nichtsulfidischen Erzen vom Salzmineraltyp durchführt.

**11.** Sulfosuccinate der Formel **(Ia)** und/oder **(Ib)**

$$R^1O-(CH_2CH_2O)_n-CO-CH_2\underset{\displaystyle SO_3M}{\underset{|}{C}}H-COOM \qquad (Ia)$$

$$R^2CO-O-(CH_2CH_2O)_m-CO-CH_2\underset{\displaystyle SO_3M}{\underset{|}{C}}H-COOM \qquad (Ib)$$

in der

| | |
|---|---|
| $R^1$ | für einen Hydroxyalkylrest mit 6 bis 22 Kohlenstoffatomen, |
| $R^2CO$ | für einen Acylrest mit 6 bis 22 Kohlenstoffatomen, |
| n | für 0 oder Zahlen von 1 bis 10, |
| m | für Zahlen von 1 bis 10 und |
| M | für Natrium oder Kalium steht, |

**12.** Verfahren zur Herstellung von Sulfosuccinaten der Formel **(Ia)** und/oder **(Ib)**,

$$R^1O-(CH_2CH_2O)_n-CO-CH_2\underset{\displaystyle SO_3M}{\underset{|}{C}}H-COOM \qquad (Ia)$$

$$R^2CO-O-(CH_2CH_2O)_m-CO-CH_2\underset{\displaystyle SO_3M}{\underset{|}{C}}H-COOM \qquad (Ib)$$

in der

| | |
|---|---|
| $R^1$ | für einen Hydroxyalkylrest mit 6 bis 22 Kohlenstoffatomen, |
| $R^2CO$ | für einen Acylrest mit 6 bis 22 Kohlenstoffatomen, |
| n | für 0 oder Zahlen von 1 bis 10, |
| m | für Zahlen von 1 bis 10 und |
| M | für Natrium oder Kalium steht, |

**dadurch gekennzeichnet,** daß man Maleinsäureanhydrid mit Hydroxyalkoholen bzw. deren (Poly-)ethylenglycolethern der Formel **(Ic)** und/oder Fettsäure(poly)ethylenglycolestern der Formel **(Id)**,

$$R^1O-(CH_2CH_2O)_nH \qquad (Ic)$$

$$R^2CO-O-(CH_2CH_2O)_mH \qquad (Id)$$

in der $R^1$, $R^2CO$, n und m die oben angegebene Bedeutung besitzen, zu den entsprechenden Maleinsäurehalbestern umsetzt und an diese in an sich bekannter Weise Natrium- oder Kaliumbisulfit anlagert.

Europäisches Patentamt

# EUROPÄISCHER RECHERCHENBERICHT

Nummer der Anmeldung

EP 92 11 9697

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | DE-A-4 010 911 (BEROL NOBEL AB)<br>* das ganze Dokument *<br>--- | 1,5,6,8, 10,11 | C07C309/17<br>B03D1/00 |
| Y | US-A-4 139 481 (S.S. WANG ET AL.)<br>* Spalte 5 - Spalte 6; Ansprüche *<br>* Spalte 4 - Spalte 5; Beispiel *<br>--- | 1,5,6,8, 10,11 | |
| D,Y | EP-A-0 067 137 (BEROL KEMI AB)<br>siehe das ganze Dokument; im besonderen Seite 5, Beispiel 1<br>--- | 1,5,10, 11 | |
| Y | US-A-3 447 681 (J.L. RAMIREZ)<br>* das ganze Dokument *<br>--- | 1,5,10, 11 | |
| D,A | AUFBEREITUNGSTECHNIK<br>Bd. 26, Nr. 11, 1985,<br>Seiten 632 - 639<br>W. VON RYBINSKI ET AL. 'Adsorption von Tensidmischungen beim Flotationsvorgang'<br>siehe das ganze Dokument; im besonderen Seite 639, Spalte 2, Tabelle 1<br>--- | 1,11 | RECHERCHIERTE SACHGEBIETE (Int. Cl.5)<br>C07C<br>B03D |
| A | US-A-4 138 350 (S.S. WANG ET AL.)<br>* Spalte 7 - Spalte 8; Ansprüche *<br>* Spalte 4 - Spalte 6; Beispiele 1,2 *<br>--- | 1,11 | |
| A | EP-A-0 270 986 (HENKEL KOMMANDITGESELLSCHAFT AUF AKTIEN)<br>* das ganze Dokument *<br>----- | 1,11 | |

Der vorliegende Recherchenbericht wurde für alle Patentansprüche erstellt

| Recherchenort | Abschlußdatum der Recherche | Prüfer |
|---|---|---|
| DEN HAAG | 22 JANUAR 1993 | FINK D.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)